# FASCICULE DE BREVET EUROPEEN

(11) **EP 0 436 420 B1**
(45) Date de publication et mention de la délivrance du brevet: **06.04.1994**
(21) Numéro de dépôt: 90403638.1
(22) Date de dépôt: 18.12.1990
(51) Int. Cl.: A61F 2/24

(54) **Valve cardiaque à clapets pivotant sur des billes**
Herzklappen mit auf Kugeln drehenden Blattelementen
Heart valve with leaflets pivoted on balls

(30) Priorité: 20.12.1989 FR 8916900
(43) Date de publication de la demande: 10.07.1991
(73) Titulaire: COMMISSARIAT A L'ENERGIE ATOMIQUE, 75015 Paris (FR)
(72) Inventeur: Boyer, Robert, F-26700 Pierrelatte (FR); Ranc, René, F-38100 Grenoble (FR); Stefani, René, F-38240 Meylan (FR)
(74) Mandataire: Mongrédien, André

(56) Documents cités:
- EP-A- 0 039 217
- EP-A- 0 091 746
- BE-A- 678 968
- RCA TECHNICAL NOTES, no. 1151, 24 mai 1976, pages 1-4; J.W. KNOLL: "Prosthetic heart valve"
- IDEM

## Description

L'invention concerne une valve cardiaque à clapets pivotant conformément au préambule de la revendication 1. Des telles valves sont connues par exemple du document EP-A-0 091 746.

On utilise maintenant couramment des valves implantées sur le muscle cardiaque qui permettent de remplacer des valves naturelles défaillantes. Ces valves comprennent d'une façon générale un anneau de maintien de la valve sur le muscle et qui porte un moyen d'articulation d'un ou plusieurs clapets qui se déplacent sous l'action du flux sanguin entre une position d'ouverture correspondant au sens d'écoulement souhaité et une position de fermeture correspondant au sens inverse. Les qualités principales que devraient présenter de telles valves sont essentiellement une bonne étanchéité en position de fermeture, la création d'une faible perte de charge dans le sens de l'ouverture, une usure lente et l'inaptitude à susciter des coagulations et des thromboses.

Les valves existantes ne permettent d'atteindre qu'imparfaitement tous ces objectifs. Parmi les systèmes existant à ce jour, on peut citer tout d'abord les valves dans lesquelles le clapet se déplace par des translations alternatives. Le système de liaison à l'anneau est alors une armature constituée d'une tige recourbée de façon appropriée, et le clapet peut être constitué par une bille ou par un disque. Ces valves créent une perte de charge notable dans le sens de l'ouverture car le clapet continue à perturber le flux sanguin, et elles sont par ailleurs sujettes au frottement et à l'usure. C'est pourquoi on a développé des valves à un ou deux clapets reliés à l'anneau par des pivots matérialisant des axes de rotation excentrés, de sorte que la pression du sang suffit à les faire pivoter. Toutefois une usure subsiste, ainsi que des concentrations de contraintes sur les pivots et les parties voisines des clapets et de l'anneau. De plus, le montage de telles valves n'est pas aisé car il faut créer des déformations élastiques pour permettre l'insertion des pivots dans une cavité des clapets ou de l'anneau. Une telle opération présente le risque de détériorer le revêtement de matériaux anticoagulants, tels que le pyrocarbone, qui recouvrent normalement le matériau de substrat des différentes pièces.

L'objet de l'invention est donc d'éviter ces inconvénients et d'offrir une valve cardiaque dans laquelle les frottements et l'usure sont réduits, le montage facile et le fonctionnement aisé.

Cette valve comprend généralement un anneau, au moins un clapet entouré par l'anneau, des moyens de pivotement permettant de faire pivoter les clapets par rapport à l'anneau, des moyens de butée pour les clapets qui délimitent une course de pivotement des clapets entre une position d'ouverture et une position de fermeture de la valve, et se caractérise en ce que les moyens de pivotement sont constitués par deux billes pour chaque clapet, et en ce que l'anneau et les clapets sont pourvus de cavités, chaque bille étant logée partiellement dans une cavité d'anneau et partiellement dans une cavité de clapet. L'anneau est par ailleurs constitué d'une bague interne et d'une bague externe concentriques et jointives, les cavités d'anneau étant portées par la bague interne exclusivement et traversant radialement la bague interne de part en part.

De préférence, les cavités des clapets ont un fond conique.

On va maintenant décrire deux réalisations de l'invention à l'aide des figures suivantes annexées à titre illustratif et non limitatif :
- les figures 1 et 2 représentent une réalisation de l'invention à clapet unique en coupe longitudinale et en vue de dessus ; et
- les figures 3 et 4 représentent des vues similaires d'une autre réalisation de l'invention à double clapet.

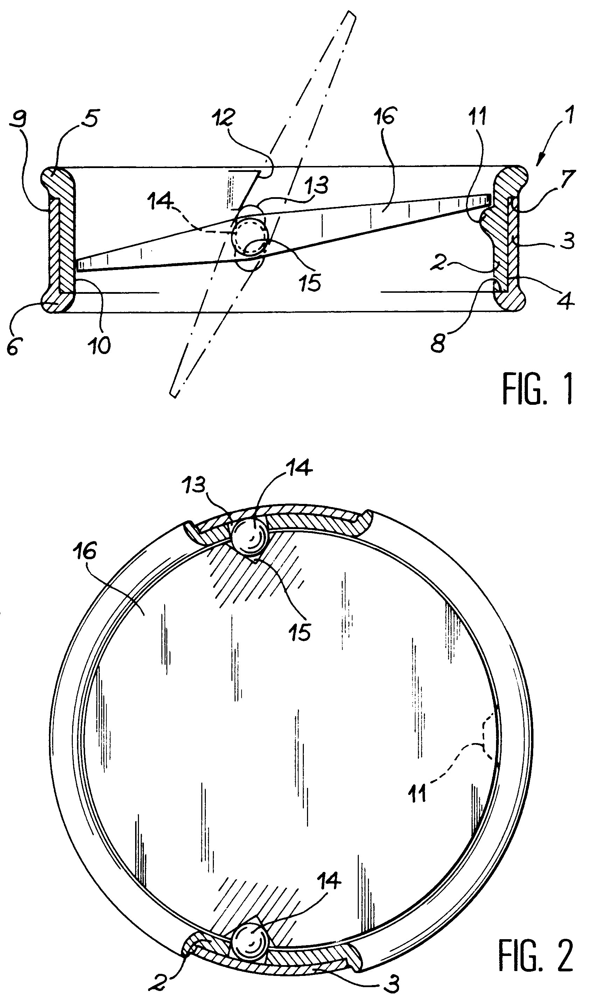

La valve des figures 1 et 2 comporte donc un anneau 1 constitué d'une bague interne 2 et d'une bague externe 3 qui sont concentriques et jointives par une surface de liaison cylindrique 4. Chacune des bagues interne 2 et externe 3 comprend un bourrelet circonférentiel orienté vers l'extérieur, respectivement 5 et 6, chacun des bourrelets étant terminé par une surface, respectivement 7 et 8, sur laquelle une portée d'extrémité de l'autre bague vient buter. On voit qu'il est possible d'assurer l'assemblage de l'anneau 1 en plaçant d'abord les bagues interne 2 et externe 3 en prolongement puis en les faisant coulisser l'une dans l'autre jusqu'à ce que la butée se produise aux surfaces 7 et 8. Après ce sertissage, la liaison peut être rendue irréversible au moyen d'une soudure ou de tout autre moyen équivalent sur les lignes de joint. Les bourrelets 5 et 6 sont alors situés aux extrémités longitudinales de l'anneau 1, et la face extérieure 9 est en dépression entre eux.

La bague externe 3 est lisse et continue, c'est-à-dire parfaitement cylindrique, en dehors du bourrelet 6; par contre, la bague interne 2 est munie sur sa face interne 10 d'une butée 11 de position fermée et d'une butée 12 de position ouverte du clapet, ainsi que de deux cavités d'anneau 13 qui la traversent de part en part et dont l'orientation est radiale. Toutefois, les deux cavités 13 ne sont pas diamétralement opposées sur la bague interne 2.

Les cavités d'anneau 13 reçoivent chacune environ la moitié d'une bille 14 dont le reste est en grande partie contenu dans une cavité de clapet 15 de forme conique, opérée sur un clapet 16 en forme de disque. Comme on le voit sur la figure 2, le jeu radial entre le clapet 16 et la bague interne 2 est faible, et le jeu circonférentiel et radial de débattement des billes 14 dans les cavités d'anneau 13 et les cavités de clapet 15 respectives est presque inexistant. Par contre, les cavités d'anneau 13 peuvent avoir une section oblongue, plus étendue dans le sens axial de l'anneau 1, ce qui permet des débattements des billes 14 et du clapet 16 de quelques dixièmes de millimètre ou de quelques millimètres dans cette direction. Cette disposition a pour but d'éviter les coagulations en assurant un mouvement notable des pièces constitutives de la valve près de l'axe de pivotement. Le brevet français 2 331 997 décrit une conception analogue dans une valve où les pivots sont d'un bloc avec l'anneau ou le clapet.

Voici comment on peut analyser les avantages particuliers à la présente invention. Tout d'abord, la construction de l'anneau 1 à l'aide de deux bagues jointives 2 et 3, dont la première est percée de cavités d'anneau 13, permet de réaliser le montage en positionnant le clapet 16 par rapport à la bague interne 2, puis en introduisant les billes 14 dans les cavités d'anneau et de clapet 13 et 15, et enfin en réalisant le sertissage des deux bagues. A aucun moment on ne crée de déformations importantes. Par ailleurs, la forme sphérique des billes 14 limite les surfaces de frottement et donc l'importance de celui-ci, ce qui épargne des efforts supplémentaires au muscle cardiaque. Des avantages supplémentaires de la forme sphérique sont la diminution des concentrations de contraintes subies et une répartition uniforme de l'usure par suite des rotations inévitables des billes 14 dans leurs cavités, selon des mouvements aléatoires et indépendants des mouvements de rotation du clapet 16, ce qui devrait permettre d'augmenter sensiblement la durée de fonctionnement envisageable de ces valves. Enfin, le fait de disposer de pivots séparés aussi bien de l'anneau 1 que du clapet 16 offre des possibilités supplémentaires de choix et de combinaison de matériaux. En fonction des matériaux éventuellement différents utilisés pour l'anneau 1 et le clapet 16, il est possible de choisir pour les billes 14 un matériau optimal du point de vue de l'usure et des frottements.

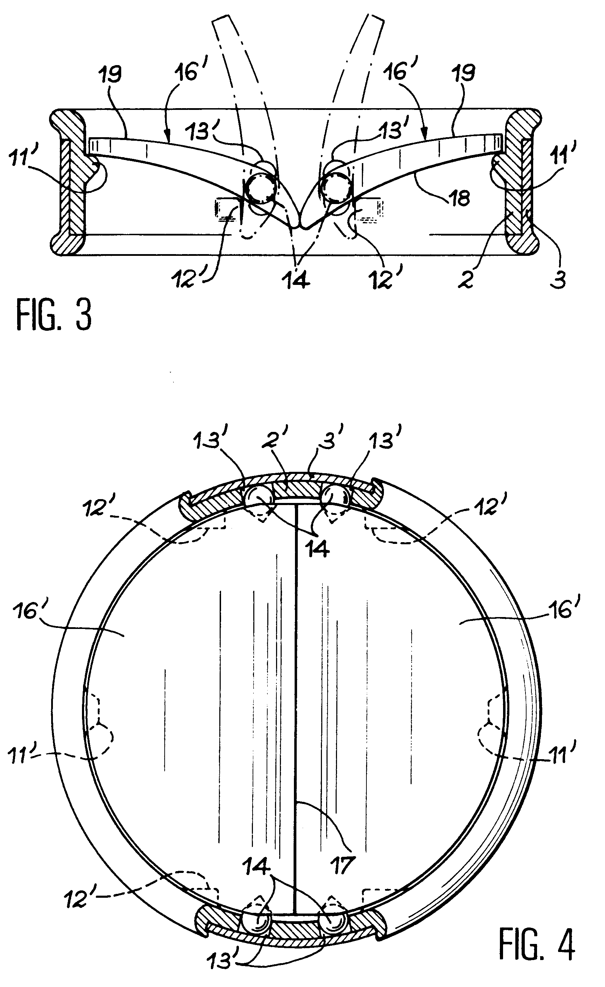

On a également représenté sur les figures 3 et 4 une autre réalisation, dans laquelle le clapet 16 unique est remplacé par deux clapets 16' de forme générale semi-circulaire. Il est alors nécessaire de modifier la bague interne maintenant référencée 2' : elle comporte désormais quatre cavités d'anneau 13' pour la réception partielle de quatre billes 14. La disposition d'ensemble est telle que chaque bille 14 est, en suivant la circonférence de la bague interne 2, proche d'une deuxième bille et diamétralement opposée à une troisième bille, ces deuxième et troisième billes servant à faire pivoter un des clapets 16'. Il en résulte que les clapets 16' pivotent autour d'axes parallèles proches l'un de l'autre ; en position de fermeture, l'étanchéité est sauvegardée car les clapets 16' se touchent sur une ligne de joint centrale 17 ; en position d'ouverture, le sang s'écoule symétriquement dans trois canaux adjacents délimités par l'anneau 1 et les clapets 16', et son écoulement est donc meilleur qu'avec la valve précédente ; cet avantage est renforcé si les clapets 16' sont courbes et présentent une face amont 18 concave et une face aval 19 convexe, alors que le clapet 16 précédent était plan tout en s'amincissant en s'éloignant de l'axe de pivotement.

Il est évidemment nécessaire de prévoir au moins une butée de position fermée 11' et une butée de position ouverte 12' pour chacun des clapets 16'. Ceci mis à part, toutes les dispositions de construction applicables à la première réalisation peuvent se retrouver ici.

## Revendications

1. Valve cardiaque comprenant un anneau (1), au moins un clapet (16, 16') entouré par l'anneau, des moyens de pivotement permettant de faire pivoter les clapets par rapport à l'anneau, des moyens de butée (11, 12, 11', 12') pour les clapets qui délimitent une course de pivotement des clapets entre une position d'ouverture et une position de fermeture de la valve, caractérisée en ce que les moyens de pivotement sont constitués par deux billes (14) pour chaque clapet et en ce que l'anneau (1) et les clapets (16, 16') sont pourvus de cavités (13, 13', 15), chaque bille (14) étant logée partiellement dans une cavité d'anneau (13, 13') et partiellement dans une cavité de clapet (15) et en ce que l'anneau est constitué d'une bague interne (2) et d'une bague externe (3) concentriques et jointives, les cavités d'anneau (13, 13') étant portées par la bague interne (2) exclusivement et traversant radialement la bague interne de part en part.

2. Valve cardiaque suivant la revendication 1, caractérisée en ce que certaines des cavités (13, 13') ont une section allongée permettant des déplacements en translation des clapets (16, 16').

3. Valve suivant l'une quelconque des revendications 1 ou 2, caractérisée en ce que les cavités de clapet (15) ont un fond conique.

4. Valve suivant l'une quelconque des revendications 1 à 3, caractérisée par un clapet unique.

5. Valve suivant l'une quelconque des revendications 1 à 3, caractérisée par deux clapets pivotant autour d'axes parallèles.

## Claims

1. Cardiac valve comprising a ring (1), at least one flap (16,16') surrounded by the ring, pivoting means making it possible to pivot the flaps with respect to the ring, abutment means (11,12,11',12') for the flaps which define a pivoting travel of the flaps between an open position and a closed position of the valve, characterized in that the pivoting means are constituted by two balls (14) for each flap and in that the ring (1) and the flaps (16,16') are provided with cavities (13,13',15), each ball (14) being partly located in a ring cavity (13,13') and partly in a flap cavity (15) and in that the ring is constituted by an inner member (2) and an outer member (3), which are concentric and contiguous, the ring cavities (13,13') being carried exclusively by the inner member (2) and radially traverse the inner member.

2. Cardiac valve according to claim 1, characterized in that certain of the cavities (13,13') have an elongated section permitting displacements in translation of the flaps (16,16').

3. Valve according to either of the claims 1 and 2, characterized in that the flap cavity (15) have a conical bottom.

4. Valve according to any one of the claims 1 to 3, characterized by a single flap.

5. Valve according to any one of the claims 1 to 3, characterized by two flaps pivoting about parallel axes.

## Patentansprüche

1. Herzventil, umfassend einen Ring (1), wenigstens eine Klappe (16, 16'), umgeben von dem Ring, Schwenkeinrichtungen für das Schwenken der Klappen bezüglich des Rings, Anschlageinrichtungen (11, 12, 11', 12') für die Klappen, die einen Schwenkhub der Klappen begrenzen zwischen einer öffnungsstellung und einere Verschlußstellung des Ventils,
dadurch **gekennzeichnet**,
daß die Schwenkeinrichtungen gebildet werden durch zwei Kugeln (14) für jede Klappe und dadurch, daß der Ring (1) und die Klappen (16. 16') mit Vertiefungen (13, 13', 15) versehen sind, wobei jede Kugel (14) partiell in einer Ringvertiefung (13, 13') und partiell in einer Klappenvertiefung (15) sitzt, und dadurch, daß der Ring gebildet wird durch einen Innenring (2) und einen Außenring (3), konzentrisch und fugendicht, wobei die Ringvertiefungen ausschließlich auf dem Innenring (2) enthalten sind und den Innenring radial von einer Seite zur anderen durchqueren.

2. Ventil nach Anspruch 1, dadurch gekennzeichnet, daß einige der Vertiefungen (13, 13') einen gestreckten Querschnitt aufweisen, der Längsverschiebungen der Klappen (16, 16') erlaubt.

3. Ventil nach einem der Ansprüche 1 oder 2, dadurch gekennzeichnet, daß die Klappenvertiefungen (15) einen konischen Unterteil aufweisen.

4. Ventil nach einem der Ansprüche 1 bis 3, gekennzeichnet durch eine einzige Klappe.

5. Ventil nach einem der Ansprüche 1 bis 3, gekennzeichnet durch zwei Klappen, schwenkbar um parallele Achsen.
